Europäisches Patentamt

European Patent Office

Office européen des brevets

(11) Publication number: 0 331 469
A2

## EUROPEAN PATENT APPLICATION

(21) Application number: 89302047.9

(22) Date of filing: 01.03.89

(51) Int. Cl.4: G 02 B 5/26

(30) Priority: 04.03.88 GB 8805267

(43) Date of publication of application:
06.09.89 Bulletin 89/36

(84) Designated Contracting States: DE FR GB

(71) Applicant: GEC-Marconi Limited
The Grove Warren Lane
Stanmore Middlesex HA7 4LY (GB)

(72) Inventor: Howard, Richard Keith
8 Woodbury Road Walderslade
Chatham Kent (GB)

(74) Representative: Pope, Michael Bertram Wingate
Central Patent Department Wembley Office The General
Electric Company, p.l.c. Hirst Research Centre East Lane
Wembley Middlesex HA9 7PP (GB)

(54) Frequency selective optical filters.

(57) A frequency selective optical filter, more particularly for laser eye protection goggles, comprising: first (17) and second (23,27) meniscus lenses having negative and positive powers respectively, the concave surface (17b) of the first lens (17) conforming with the convex surface (23a) of the second lens (23,27) and the sum of the powers of the first (17) and the second (23,27) lenses equating substantially to zero; and at the conformal interface between the lenses (17 and 23,27) a diffractive mirror coating (21).

The filter increases the angular protection range afforded without increasing the spectral bandwidth and hence reduction of visible photopic transmission.

*Fig.6.*

EP 0 331 469 A2

## Description

### Frequency Selective Optical Filters.

This invention relates to frequency selective optical filters, more particularly such filters suitable for use in laser eye protection filters.

Diffractive mirrors may be used to reflect unwanted light of fixed frequency or narrow band width away from optical systems. Such mirrors form an important technique when designing and constructing laser eye protection filters. These mirrors are Bragg devices and as such are angularly dependent. A particular wavelength will only be reflected from the filter over a range of angles which is determined by both the wavelength and spectral width of the filter, according to the Bragg equation

$$\frac{\lambda}{\cos\phi} = \text{constant}$$

where $\lambda$ = wavelength of filter measured at the internal angle $\varphi$ to the normal to the mirror.

If the normal incidence wavelength at the lower wavelength edge of the filter's response is less than the desired reflection wavelength and the filter's response is sufficiently wide to encompass the desired reflection wavelength then this wavelength will be reflected over a range of angles between $0°$ and $\pm \cos^{-1}\left(\frac{\lambda_1}{\lambda_2}\right)°$,

where
$\lambda_1$ is the wavelength to be reflected and
$\lambda_2$ is the normal incidence wavelength of the high side of the filter's response.

In order to obtain a large angular protection range behind known forms of diffractive mirror filter a large spectral width is required. This has a serious impact on the integrated visible photopic transmission if the threat wavelength is in the visible light range. It is therefore an object of the present invention to provide a filter which allows a large angular protection range with a relatively narrow spectral bandwidth.

It is a further object of the present invention to provide such filters which are suitable for use in a goggles or spectacle frames which support the filters at a distance from the eye substantially the same as that encountered with corrective lenses in conventional spectacles.

According to the invention a frequency selective optical filter comprises: a first meniscus lens having a negative power, a second meniscus lens having a positive power, the first lens having a concave surface conforming with a convex surface of the second lens and the sum of the powers of the first and second lenses equating substantially to zero: and, at the conformal interface between the first and second lenses, a diffractive mirror coating.

The diffractive mirror coating is suitably a holographic coating, e.g. a photopolymer coating, but may, however, be a dielectric coating.

In a preferred form of filter according to the invention the other surface of said first lens has a convex curvature which is substantially equal to the curvature of the other surface of the second lens, which other surface of the second lens is concave.

In one particular embodiment of the invention the second lens comprises: a first light absorbent part having concentric convex and concave surfaces, said convex surface of said first part constituting the convex surface of the second lens; a second part having a convex surface which conforms with the concave surface of the first part; and, between the conformal surfaces of said first and second parts, a second diffractive mirror coating.

In such an element the second part of the second lens is suitably made of a plastics material, e.g. a polycarbonate, and the second diffractive mirror coating is suitably a dielectric coating.

The invention will now be further explained and a preferred form of filter in accordance with the invention will now be described, by way of example, with reference to the accompanying drawings, in which:

Figures 1 to 5 represent ray tracings through Bragg devices incorporated in optical filters of different sections; and

Figure 6 is a diagram depicting a section across the preferred form of optical filter in accordance with the invention.

It is a consideration of practical importance in the design of optical filters incorporating Bragg devices, being filters suitable for use as eye protection against laser damage, to determine the location of the zone of protection the zone, that is, not penetrated by laser rays, relative to the filter element.

Generally speaking it is desirable to have the protected area as near as possible to the filter substrate and as large as possible. This allows protection of either sensors or systems where the sensitive area is large, ill defined relative to the filter or close to the filter requiring a large range of working angles.

The two conditions mentioned in the preamble to this specification, that it is desirable to produce a filter design allowing large angular coverage but with relatively narrow spectral bandwidth, are mutually exclusive when considering a flat substrate on which a diffractive mirror, the Bragg device is deposited.

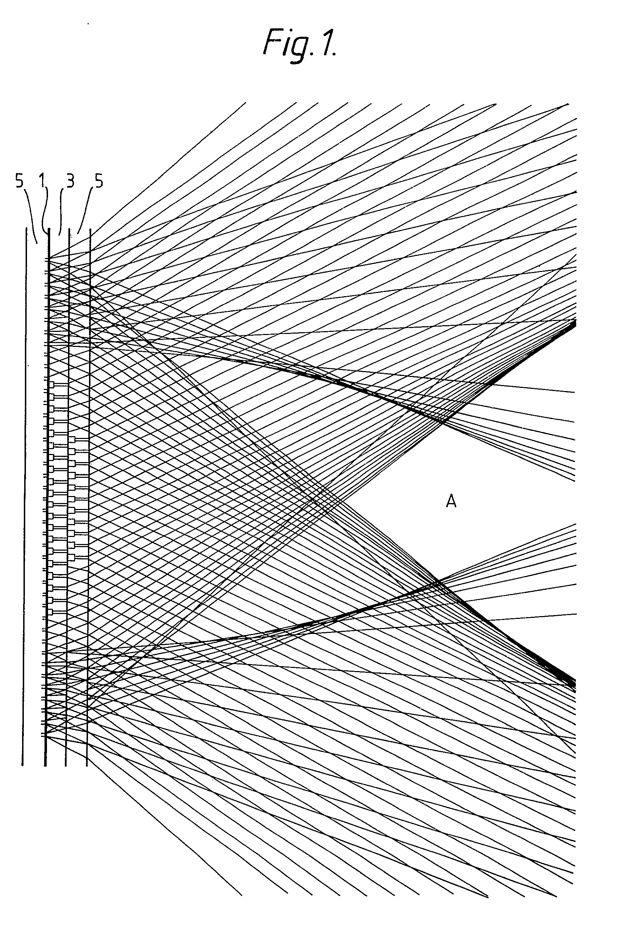

This is brought out in Figure 1 where, for a filter element comprising a flat diffractive mirror 1 and a flat plate 3 of light absorbent glass sandwiched between a pair of outer flat glass plates 5, cones from several points of the surface of the mirror 1 representing the boundraries of the reflected/transmitted rays through the filter have been plotted.

A holographic diffractive mirror 1 of 30 nm spectral width has been used in this example. The zone marked A is a zone from which threat rays (as represented) are excluded. This zone may be changed in position or shape by changing the spectral parameters of the mirror 1. However, a significant increase in width is required to make the protected zone A of a useful size.

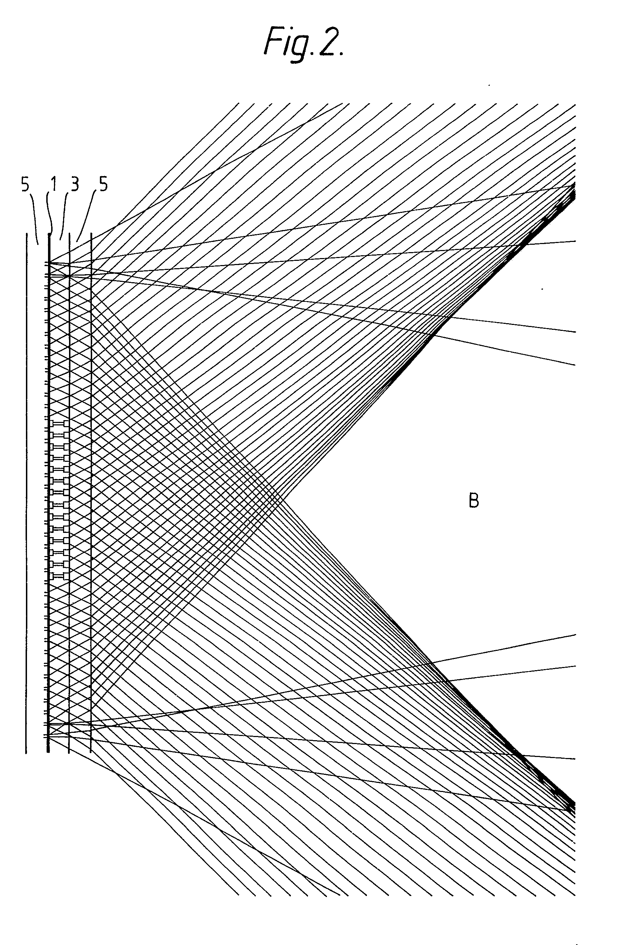

Figure 2 shows the response of the filter when the spectral width of the mirror 1 is doubled. As may be seen, the protected zone B is larger than region A in Figure 1, but the increase in width causes serious degradation in the photopic transmission of the filter element.

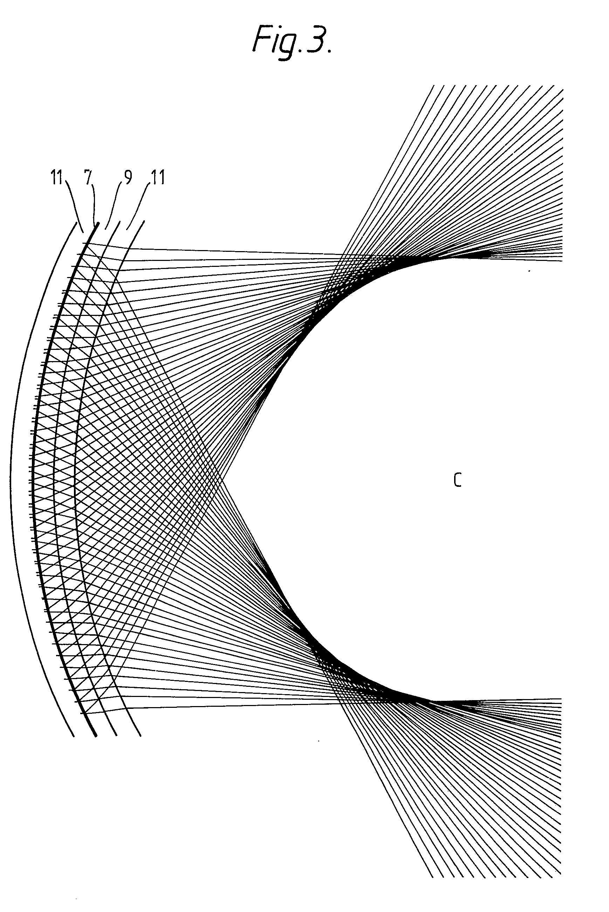

The performance may be improved by using a curved diffractive mirror 7 between similarly curved absorbent 9 and outer plates 11 such that the overall filter still has zero optical power, as seen in Figure 3. The protected C zone in this design is larger and nearer the filter than for the flat design of Figure 1, although the spectral width of the filter is the same as in Figure 1.

In many applications a large curvature in front of a sensor may be undesirable for optical reasons.

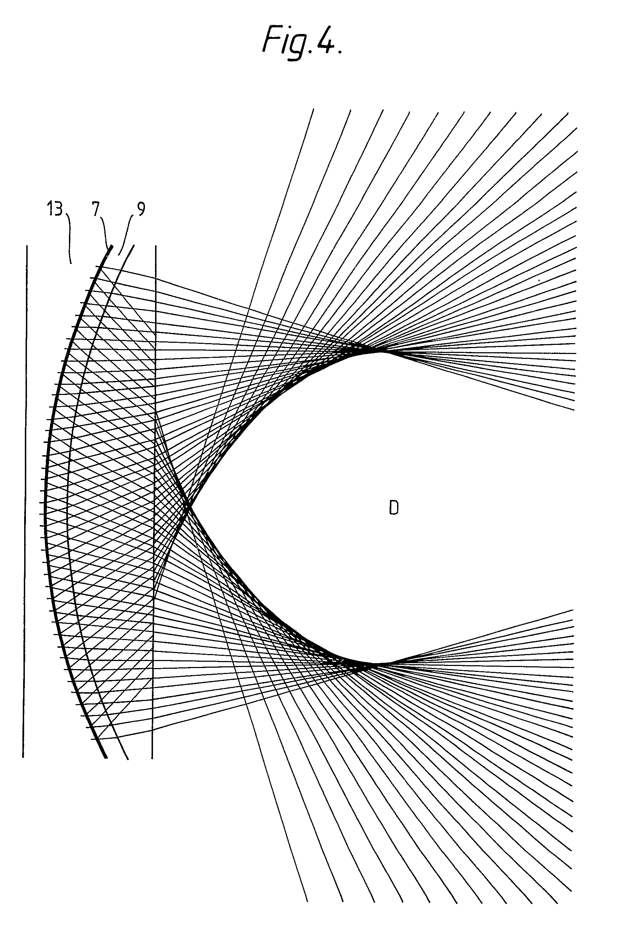

According to the present invention the design is improved by the containment of the curved mirror 7 and absorbent plate 9 within a lower curvature block of glass, or other transparent material. Figure 4 shows such a design using a 12 dioptre curved mirror 7 within a flat and parallel faced block of glass 13. The key feature here is that the zone of protection D is much closer to the filter although the size of the protected area is smaller than that seen in Figure 3. The weight of this design is, of course, much increased over the weight of the filters shown in Figure 1 or Figure 3.

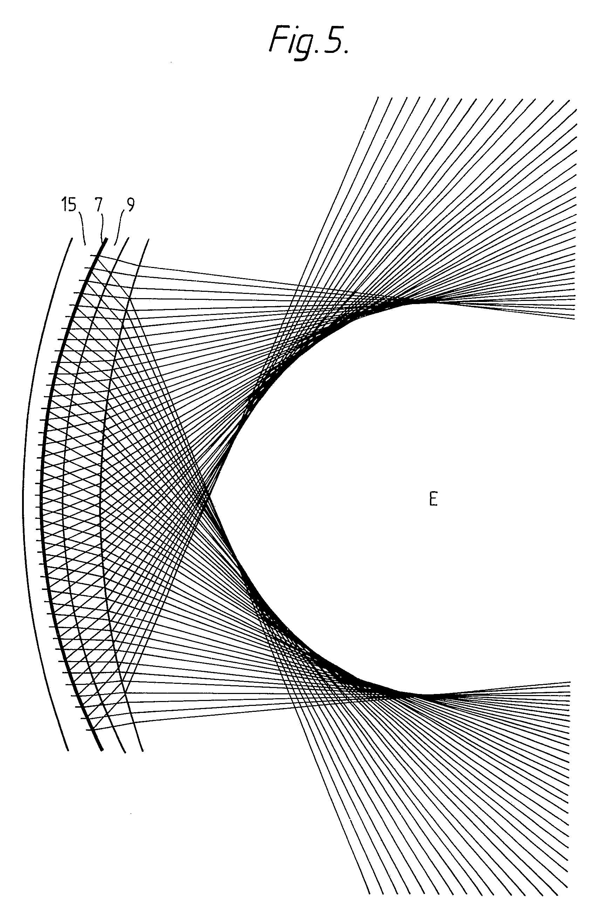

The optimum design is to incorporate the 12 dioptre curve mirror 7 and absorbent plate 9 within a 9 dioptre faced block 15 as shown in Figure 5. The protected zone E is nearer the filter than in the 12 dioptre base curve design (Figure 3) without any significant reduction in size. The weight of the filter is less than that shown in Figure 4.

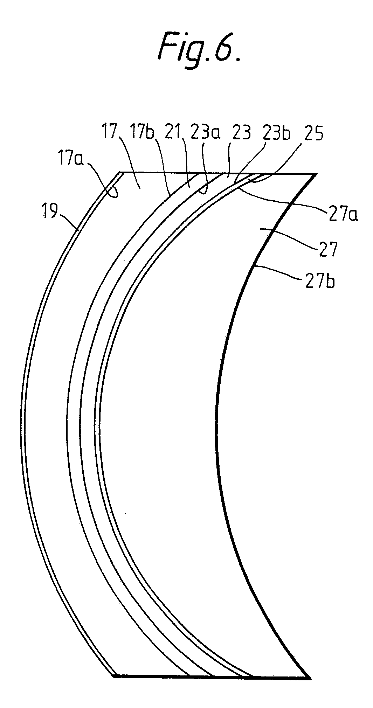

These key features are demonstracted in a practical embodiment of the laser eye protection filter, shown in Figure 6, two of which may be mounted for example, in a suitable pair of spectacle frames. The filter includes a meniscus lens outer element 17 of crown glass whose convex surface curvature 17a is of nine dioptres whilst the concave surface 17b is of twelve dioptres. An anti-reflective coating 19 is deposited on the convex surface 17a of the element 17. A diffractive coating 21, in the embodiment a holographic mirror, is deposited on the concave surface 17b of element 17.

The fringes of the holographic recording comprising the mirror 21 are conformal to the concave glass surface 17b, the holographic mirror 21 reflecting at a wavelength equal to an optical threat wavelength. This would, typically, be for a threat at the 532 nm, frequency doubled Nd Yag laser wavelength. The anti-reflective properties of the coating 19 on the outer surface 17a of the crown element 17 contribute in the construction of the hologram and in the performance of the device.

The middle element 23 is constructed from an absorbent glass such as KG3, the purpose of which is to absorb light at the Nd-Yag laser wavelength of 1.06 um. The element 23 has a convex surface 23a which matches the shape of the concave surface 17b of the crown element 17. The element 23 is of constant thickness so that its concave surface 23b has the same curvature as and is concentric with its convex surface 23a.

The convex surface 23a of element 23 is laminated onto the face of the holographic mirror 21 using an optical cement. The concave surface 23b of the element 23 supports a dielectric mirror coating 25 designed to reflect wavelengths from the 694 nm ruby wavelength up to about 830 nm which also covers the Alexandite laser wavelength.

The further filter includes a third element 27, in the form of a plastic meniscus lens made of, for example, polycarbonate or CR39, which is constructed to have equal and opposite power to the glass sub-assembly 17,23. The element 27 has convex 27a and concave 27b surfaces having the same curvature as the surfaces 17b and 17a respectively of the lens 17, the convex surface 27a being laminated onto the mirror coating 25, the three elements 17,23,27 together thus having substantially zero power. The plastic element 27 is beneficial also in reducing the wieght of the total filter (compared with an all glass construction) providing a significant degree of ballistic protection for the user and additionally providing optical attenuation of wavelengths below about 380 nm.

The concave surface 27b of the plastic element 27 may be provided with an anti-reflective coating (not shown) to reduce transmission losses yet further.

The filter of Figure 6 provides optical protection against laser threats, using a hybrid of holographic mirror, dielectric mirror and absorbing glass technologies incorporated in a novel configuration providing optimum performance for the working requirements of the filter, which needs to cater for a large range of possible eye positions.

The diffractive coating 21 is preferably a reflection hologram recorded in a suitable recording medium. However, a multi layer dielectric stack evaporated onto the surface 17b may however be employed instead.

In summary, features of the design of the filter of Figure 6 are:-

A. Use of a high curvature surface 17b in order to reduce the spectral width of the mirror coating 21 required to give a large protection zone.

B. The embedding of this surface 17b within a shallower substrate (17,27) in order to increase the angular coverage of the mirror 21 and move the protection zone nearer to the filter. This works by using the refraction at the lens 27/air interface to advantage and refracting the rays away from the protected zone thus increasing its size.

C. The splitting of the filter into three parts 17,23,27 the inner element 27 being made of plastic so as to reduce the overall weight and provide some impact protection in the overall filter.

D. The provision within the filter of a parallel surface element 23 which may be made of an absorbing glass or plastic, its constant thickness giving uniform attenuation over the surface of the filter.

E. The provision within the filter of two internal concave surfaces 17b and 23a on which dielectric coatings may be deposited, the properties of such coatings being more uniform when their deposition is on a concave surface.

F. The provision within the filter of a concave 17b and a convex surface 23a, upon which holograms may be deposited and which are subsequently laminated together forming a rugged environmental seal against moisture. This is of particular importance when dichromated gelatin is employed as a recording medium of the hologram.

## Claims

1. A frequency selective optical filter comprising: a first meniscus lens (17) having a negative power, a second meniscus lens (23,27) having a positive power, the first lens having a concave surface (17b) conforming with a convex surface (23a) of the second lens (23,27) and the sum of the powers of the first (17) and second (23,27) lenses equating substantially to zero; and, at the conformal interface between the first (17) and second (23,27) lenses, a diffractive mirror coating (21).

2. A filter according to Claim 1, wherein said coating (21) is a holographic coating.

3. A filter according to Claim 1, wherein said coating (21) is a dielectric coating.

4. A filter according to Claim 1 or Claim 2 or Claim 3, wherein the other surface (17a) of said first lens (17) has a convex curvature which is substantially equal to the curvature of the other surface (27b) of the second lens (23,27), which other surface (27b) of the second lens (27) is concave.

5. A filter according to Claim 4, wherein said concave surface (17b) of said first lens (17) and said convex surface (23a) of said second lens (23,27) have a 12 dioptre curvature and said other surfaces (17a,27b) of said first (17) and second (23,27) lenses have a 9 dioptre curvature.

6. A filter according to any one of the preceding claims wherein said second lens (23,27) comprises: a first light absorbent part (23) having concentric convex (23a) and concave (23b) surfaces, said convex surface (23a) of said first part (23) constituting the convex surface (23a) of the second lens (23,27); a second part (27) having a convex surface (27a) which conforms with the concave surface (23b) of the first part (23); and, between the conformal surfaces (23b,27a) of said first (23) and second (27) parts, a second diffractive mirror coating (25).

7. A filter according to Claim 6 wherein said second part (27) is made of a plastics material.

8. A filter according to Claim 7, wherein said plastics material is a polycarbonate material.

9. A filter according to Claim 6, 7 or 8, wherein said second diffractive mirror coating (25) is a dielectric coating.

10. A laser eye protection filter comprising a filter according to any one of the preceding claims.

EP  0 331 469  A2

Fig.1.

A

# Fig.2.

## Fig.3.

Fig.4.

Fig.5.

Fig.6.